(19) **Europäisches Patentamt**

**European Patent Office**

**Office européen des brevets**

(11) **EP 1 104 679 A2**

(12) **EUROPÄISCHE PATENTANMELDUNG**

| | |
|---|---|
| (43) Veröffentlichungstag:<br>**06.06.2001 Patentblatt 2001/23** | (51) Int Cl.⁷: **A61L 15/18**, A61L 15/46 |

(21) Anmeldenummer: **00124698.2**

(22) Anmeldetag: **11.11.2000**

| | |
|---|---|
| (84) Benannte Vertragsstaaten:<br>**AT BE CH CY DE DK ES FI FR GB GR IE IT LI LU<br>MC NL PT SE TR**<br>Benannte Erstreckungsstaaten:<br>**AL LT LV MK RO SI**<br><br>(30) Priorität: **03.12.1999 DE 19958458**<br><br>(71) Anmelder: **Beiersdorf Aktiengesellschaft<br>20245 Hamburg (DE)** | (72) Erfinder:<br>• **Schink, Michael, Dr.<br>22605 Hamburg (DE)**<br>• **Meyer-Ingold, Wolfgang, Dr.<br>22457 Hamburg (DE)**<br>• **Bogdahn, Michael, Dr.<br>21614 Buxtehude-Neukloster (DE)**<br>• **Ettner, Norbert, Dr.<br>86551 Aichach (DE)** |

(54) **Antimikrobiell ausgerüstete Wundauflagen**

(57)  Wundauflage, dadurch gekennzeichnet, daß sie aus einem synthetischen Polymermaterial besteht, das metallionenhaltige Zeolithe enthält.

EP 1 104 679 A2

**Beschreibung**

Die Erfindung betrifft Wundauflagen, die zur Behandlung infizierter Wunden oder zum vorbeugenden Schutz vor Wundinfektionen eingesetzt werden können.

Die Behandlung und Heilung von bakteriell kontaminierten beziehungsweise von infizierten Wunden stellt eine große Herausforderung für die Medizin und Naturwissenschaften dar. Vor allem bei schwerheilenden und chronischen Wunden tritt oftmals eine Besiedelung durch verschiedenste Mikroorganismen auf, die den Verlauf der Heilung stark verzögern oder manchmal auch völlig unterbinden. Aber auch bei akuten Wunden, die durch Traumata, chirurgische Eingriffe oder auch nur einfache Verletzungen entstanden sind, ist ein Eindringen von pathogenen Keimen nicht in jedem Fall auszuschließen.

Dadurch findet eine Kolonisation der Wunde mit Mikroorganismen statt. Bei einer Besiedlung der Wunde mit mehr als $10^5$ KBE/g spricht man von einer infizierten Wunde (M.C.Robson ,,Clinical Research can improve the outcome of treatment of problem wounds : Infection as a paradigm", 8$^{th}$ Annual Meeting of the ETRS, Copenhagen, DK, August 27-30, 1998). Durch die massive Besiedlung des Wundmilieus mit Mikroorganismen kann es zu einer massiven Störung des Heilungsverlaufes kommen, der in letzter Konsequenz zur Lethalität führen kann. Häufige Auslöser von bakteriellen Wundinfektionen gehören zu den Gattungen Pseudomonas, Staphylococcus, Clostridium und bei den Hefen und Schimmelpilzen zu den Gattungen Candida und Aspergillus. Eine Eingrenzung auf wenige Arten ist nicht möglich, da viele der Mikroorganismen als opportunistische Krankheitserreger anzusehen sind.

Sehr verschiedene Möglichkeiten sind beschrieben, Mikroorganismen aus dem kontaminierten oder infizierten Gewebe einer Wunde zu entfernen beziehungsweise darin abzutöten. Außer durch die orale Gabe von Antibiotika kann das Entfernen von pathogenen Mikroorganismen aus einer Wunde nach dem Stand der Technik durch die topische Anwendung eines Desinfektionsmittels oder eines Antibiotikums erreicht werden. Ferner sind Antiseptika und Antibiotika zytotoxisch, und darüber hinaus haben viele pathogene Stämme Resistenzen gegen Antibiotika entwickelt. Daß sogar auch Resistenz-Entwicklung gegenüber einem Antiseptikum möglich ist, wurde am Beispiel Triclosan resistenter E. coli Bakterien berichtet (McMurry LM et al (1998) FEMS Microbiol Lett 166(2): 305-9, Cookson BD et al (1991) Lancet 337 (8756): 1548-9; Uhl S (1993) Lancet 342(8865): 248). Maßgeblich dabei war vor allem der weitverbreitete und prophylaktische Einsatz von Triclosan (Irgasan®) in Seifen, Deos, Textilien und Kunststoffen.

Dieses zeigt, daß ein Bedarf neuer Therapieformen zur Behandlung infizierter Wunden besteht.

Eine andere, aber sehr aufwendige Möglichkeit ist die mechanische Reinigung der infizierten Wunde mit steriler Ringer-Lösung oder anderen Flüssigkeiten. Nachteilig dazu kann aufgeführt werden, daß dieser Vorgang häufig wiederholt werden muß, was die Heilung der Wunde verzögern kann.

Die Ringer-Lösung ist gemäß Römpp Lexikon Chemie (Version 1.5, Stuttgart/New York: Georg Thieme Verlag 1998) eine von dem Londoner Pharmakologen S. Ringer (1835-1910) angegebene isotonische Lösung, deren osmotischer Druck dem des normalen Blutes gleicht (7,55 bar). Die wäßrige Lösung enthält 0,8% Kochsalz, 0,02% Kaliumchlorid, 0,02% Calciumchlorid und 0,1% Natriumhydrogencarbonat. Die Ringer-Lösung enthält die Salze ungefähr im gleichen Verhältnis wie das Blutserum, weshalb man in ihr viele Zellen längere Zeit am Leben erhalten kann. Sie dient insbesondere als Blutersatzmittel und Infusionslösung bei Elektrolyt- und Wasserverlusten.

Hinlänglich bekannt und beispielhaft zur antimikrobiellen und/oder vorbeugenden Therapie von kontaminierten beziehungsweise infizierten Wunden ist die Verwendung von Oxidantien (zum Beispiel Jodtinktur) oder Antiseptika (zum Beispiel Salben mit Silbersulfadiazin).

Auch in Form von entsprechend antimikrobiell ausgerüsteten oder imprägnierten Wundauflagen und Wundversorgungsmaterialien kommen derartige Agentien zum Einsatz. So werden in der JP 03 083 905 Fasern, Filme, Papiere und Kunststoffe mit silberhaltigen Phosphaten beschrieben, die bakterizide und fungizide Eigenschaften aufweisen. Nachteilig gestaltet sich dabei, daß die Wunde unter einer solchen Behandlung üblicherweise austrocknet. Zwar stellt Wundexsudat ein ideales Nährmedium für Bakterien dar, und eine Reduzierung der Menge an Wundexsudat durch feuchtigkeitsaufnehmende Wundauflagen zeigt auch eine Verminderung des Bakterienwachstums.

Neben der Applikation von antimikrobiellen Zubereitungen und die Verwendung imprägnierter Wundversorgungsmaterialien wird auch der Einsatz von hydrophobierten Trägermaterialien beschrieben (EP 0 021 230 B1, EP 0 162 026 B1, EP 296 441 A1). In einem hydrophilen Milieu (Wasser, Salzlösung, Wundflüssigkeit) werden hier hydrophobe Bakterien von einer Wundauflage adsorbiert, die durch einen aufwendigen chemischen Prozeß hydrophobiert wurde. Die Bakterien werden dann durch Entfemen der Wundauflage der Wunde entzogen. Entscheidender Nachteil ist dabei, daß im Gegensatz zu den oben aufgeführten, gängigen Behandlungsmethoden Bakterien und Mikroorganismen nicht abgetötet werden. Dieser Nachteil wird noch verstärkt, wenn die behandelte Wunde austrocknet. Dies bedeutet den Verlust des hydrophilen Milieus, das entscheidend zur Wechselwirkung zwischen Wundauflage und Bakterien beiträgt. Die nicht abgetöteten Bakterien und Mikroorganismen lösen sich von der Wundauflage und fallen zurück ins Wundbett.

Es ist nach dem Stand der Technik festzustellen, daß eine trockene Wundbehandlung obsolet ist.

Die heutigen Anforderungen an die Funktion von modernen, als interaktiv bezeichneten Wundauflagen gehen zurück auf G. Winter (1962, Nature 193, 293) und sind von T. D. Turner neu formuliert (1994, Wound Rep. Reg. 2, 202). Im Vordergrund steht dabei die Schaffung eines feuchten Wundmilieus, das im Gegensatz zur traditionellen trockenen Wundbehandlung wie zum Beispiel mittels Mullkompressen den natürlichen Abläufen der Wundheilung physiologische und damit bessere Konditionen bietet.

Ein in diesem Sinne modernes Wundversorgungsprodukt ist Arglaes®, ein von Maersk Medical entwickeltes Filmdressing mit antimikrobiellen Eigenschaften. Der Wirkmechanismus von Arglaes® wird auf eine neue Technologie, genannt ,,Slow Release Polymer", zurückgeführt, die im feuchten Milieu der Wunde eine langsame, aber konstante Freisetzung von Silberionen bewirkt (Biomed. Mat. Nov. 1995; Health Industry Today, 1 Nov. 1997, Vol. 58, No.11). Diese Freisetzung führt aber letztlich auch zu direktem Kontakt von Silberionen mit Wundgewebe und damit zu der Gefahr, auch gesundes Zellwachstum während der Wundheilung zu beeinträchtigen.

In Japan wurden Zeolithpartikel entwickelt, die Silberionen enthalten. So berichtet die JP 60 181 002 über natürliche und synthetische Zeolithe, die Silber, Kupfer oder Zink enthalten und eine lang anhaltende fungizide Wirksamkeit aufweisen. Solche anorganischen Alumosilikate werden im wäßrigen Milieu durch einen Ionenaustauschmechanismus mit einer konstanten Freisetzung von Metallionen antibakteriell oder fungizid wirksam und können zum Beispiel als antibakterielles Keramikpulver mit der Bezeichnung Bactekiller® in Fasern eingearbeitet werden. Diese kommen seit langem im Haushalts- und Sanitärbereich in Form von Luftfiltern, Wandtapeten, Teppichen, Tüchern o. ä. zum Einsatz. Besonders hervorzuheben sind Zeolithpartikel, die neben Silber-Ionen auch Zink-Ionen enthalten. Auch Zink-Ionen weisen insbesondere in Kombination mit Silber-Ionen eine antibakterielle Wirkung auf (Keefer et al, Wounds 10 (1998) 54-58).

In der JP 10 120 518 werden antimikrobielle Mittel beschrieben, die als anorganische Pulver vorliegen und metallische Silberteilchen mit einer Teilchengröße von höchstens 10 nm enthalten. Auch derartige Mittel sind beispielsweise Alumosilikate (Zeolithe), die ein Einschlußgitter aufweisen und sich durch Stabilität gegen Anfärben und Umfärben durch Licht, Wärme, Druck und chemische Substanzen auszeichnen sowie eine anhaltende antimikrobielle Wirksamkeit aufweisen.

Die JP 08 294 527 beschreibt die Herstellung von auf Polyvinylalkohol basierenden Wundauflagen mit Silber enthaltenden antimikrobiellen Wirksubstanzen, wobei die Herstellung über den Weg der Gefriertrocknung aus Lösung erfolgt. Die Wundauflagen zeigen gute Biokompatibilität, Feuchtigkeits- und Sauerstoffdurchlässigkeit und eine lang anhaltende Wirkung.

In der JP 07 157 957 werden antibakterielle Polyurethanfaser und die Herstellung antibakterieller Vliese durch Spinnen aus der Schmelze veröffentlicht. Dabei werden aromatische thermoplastische Polyurethane auf Basis MDI-Polytetramethylenglycol-Blockcopolymer und silberionenhaltige Phosphorsäuresalze (Novaron AG-300) verwendet.

Die Verwendung von Zeolithen als Füllstoff ist beispielsweise in der EP 0 057 839 B1 genannt.

Die US 5,753,251 beschreibt antimikrobiell wirkende Beschichtungen, die auf einem Medizinprodukt durch Abscheidung von Metallen, beispielsweise Silber, aus der Gasphase hergestellt werden. Der antimikrobielle Effekt beruht auf einer Freisetzung von Ionen, Atomen, Molekülen oder Clustern aus einem gestörten Metallgitterverbund im Kontakt mit wasser- oder alkoholbasiertem Elektrolyt.

In WO 91/11206 werden Alginate für den Einsatz als Wundauflagen beschrieben, die Kationen aus der Gruppe Zink, Kupfer, Silber, Cer, Magnesium, Cobalt, Mangan oder Eisen enthalten. Über die Freisetzung der Metallionen aus den Alginaten und die Wirkungsweise werden hier keine Angaben gemacht.

Auch aus der WO 92/22285 sind Alginate in Kombination mit Calcium-, Magnesium-, Zink- oder Silberverbindungen, bevorzugt Silbersulfadiazin, bekannt. Beschrieben wird die Verwendung dieser Alginate in der Wundheilung. Es ist nicht beschrieben, ob eine kontrollierte Freisetzung der Metallionen aus den Alginaten erfolgt.

Aus der DE 196 31 421 A1 ist die Kombination eines hydrophoben und damit bakterienadsorbierenden Materials und eines antimikrobiellen Wirkstoffs, der nicht in die Wunde abgegeben wird, bekannt. Diese Kombination führt zu einem neuen Wirkmechanismus mit synergetischem Effekt.

Die Wundauflage dient als Barriere für Mikroorganismen und sie adsorbiert die Bakterien aus der Wundflüssigkeit. Nach der Adsorption werden diese an der Wundauflage abgetötet und mit dem Entfernen der Auflage werden die abgetöteten Bakterien und unverbrauchter Wirkstoff ebenfalls entfernt. Sie stören damit nicht mehr den Heilungsverlauf. Geeignete bakterienadsorbierende, hydrophobe Materialien können synthetische oder natürliche oder chemisch modifizierte natürliche Polymere sein wie Polyethylen, Polypropylen, Polyurethan, Polyamid, Polyester, Polyvinylchlorid, Polytetrafluorethylen oder durch kovalente Verknüpfung hydrophiler Substanzen mit hydrophoben Gruppen hergestellte Polymere sein, beispielsweise gemäß EP 0 021 230 B1. Die bakterienadsorbierenden Eigenschaften hydrophober Materialien sind bekannt (vgl. D.F. Gerson et al., Biochim. Biophys. Acta, 602 (1980, 506-510); Y. Fujioka-Hirai. et al., J. of Biochemical Materials Research, Vol. 21, 913-20 (1987); S. Hjerten et al., J. of Chromatography 101 (1974), 281-288; M. Fletcher et al., Appl. and Environmental Mikrobiology, Jan. 1979, 67-72). Die hydrophoben Eigenschaften sind auch einfach durch einen Wassertropfentest nachzuweisen, bei dem das Wasser vom Material abperlt.

Geeignete antimikrobielle Wirkstoffe, unter denen in erster Linie an sich bekannte Substanzen zu verstehen sind, wie zum Beispiel Chlorhexidin oder Phenolderivate wie Thymol und Eugenol oder die in der DE 32 15 134 C2 benannten Chlorphenole oder Chlordiphenylether, zeichnen sich dadurch aus, daß sie fest an der Wundauflage haften, an oder in dieser auf die Mikroorganismen einwirken und nicht oder zumindest nicht merklich in die Wunde abgegeben werden. Dies kann durch physikalische Ein- oder Anlagerung an geeignete Träger erfolgen, zum Beispiel Einlagerung hydrophober Wirkstoffe in hydrophobe Trägermaterialien, oder beispielsweise auch durch kovalente Bindung an diese. Die Wirkstoff/Träger-Systeme sollen sich dadurch auszeichnen, daß auch bei mehreren Extraktionen mit wäßrigen Lösungen oder Wundflüssigkeit ihre antimikrobielle Wirksamkeit erhalten bleibt. Die Wundauflagen sollten den antimikrobiellen Wirkstoff in einer Menge von mindestens 0,001 Gew.-% enthalten, um eine ausreichende Wirksamkeit zu erzielen.

Das Ziel der Erfindung ist es, eine Wundauflage zu entwickeln, die eine verbesserte Behandlung infizierter Wunden und/oder Schutz vor Infektionen ermöglicht und die die aus dem Stande der Technik bekannten Nachteile nicht aufweist.

Gelöst wird diese Aufgabe durch eine Wundauflage, wie sie im Hauptanspruch niedergelegt ist. Gegenstand der Unteransprüche sind dabei vorteilhafte Weiterbildungen der Wundauflage.

Gegenstand der Erfindung sind Wundauflagen mit antimikrobiellen Eigenschaften, dadurch gekennzeichnet, daß selbstklebende oder nicht selbstklebende Materialien, die in der Wundheilung Verwendung finden, wie synthetische Polymermaterialien, zum Beispiel Polyurethane, Polyacrylate, SIBS-Massen, SEBS-Massen, Naturkautschukmassen sowie Chitosane, Alginate, Hydrogele, Hydrokolloide, insbesondere aber Polyurethane, kombiniert werden mit silberhaltigen Zeolithen, die in bevorzugten Ausführungsformen der Erfindung mit 0,01 bis 40 Gew.-%, insbesondere bevorzugt 0,1 bis 6 Gew.-%, in die Polymermaterialien eingearbeitet werden können.

Die Bezeichnung Zeolithe ist von dem schwedischen Mineralogen Cronstedt 1756 eingeführt worden und beschreibt eine weitverbreitete Gruppe von kristallinen Silicaten, und zwar von wasserhaltigen Alkali- bzw. Erdalkali-Alumosilicaten (ähnlich den Feldspäten) der allgemeinen Formel, $XM_{2/n} \cdot O \cdot Al_2O_3 \cdot YSiO_2 \cdot ZH_2O$ (M: ein-, zwei- oder mehrwertige Metallionen wie beispielsweise $Ag^+$, $Na^+$, $Zn^{2+}$ etc.; n: Wertigkeit; X,Y,Z: partielle molare Menge, wobei folgende Richtwerte gelten: Y=1,8 bis ca. 12, Z=0 bis ca. 8) (Quelle: Römpp Lexikon Chemie - Version 1.5, Stuttgart/New York: Georg Thieme Verlag 1998).

Die Kristallgitter der Zeolithe bauen sich aus $SiO_4$- und $AlO_4$-Tetraedern auf, die über Sauerstoff-Brücken verknüpft sind. Dabei entsteht eine räumliche Anordnung gleichgebauter (Adsorptions-)Hohlräume, die über - untereinander gleich große - Fenster (Porenöffnungen) beziehungsweise Kanäle zugänglich sind. Im folgenden ist ein synthetischer Zeolith A dargestellt.

Ein derartiges Kristallgitter vermag gleichsam als Sieb zu wirken, welches Moleküle mit kleinerem Querschnitt als die Porenöffnungen in die Hohlräume des Gitters aufnimmt, während größere Moleküle nicht eindringen können (sogenannte Molekularsiebe).

Die zum Ausgleich der negativen Ladung der $AlO_4$-Tetraeder im Alumosilicatgerüst notwendigen Kationen sind im hydratisierten Gitter relativ beweglich und können leicht gegen andere Metall-Ionen ausgetauscht werden, was die Ionenaustauscher-Eigenschaften bedingt, so mindern in Waschmitteln die Zeolithe (insbesondere das Zeolith A) die Härte des Wassers, weil sie die Calcium-Ionen aus dem Wasser und den Anschmutzungen entfernen.

Eine weitere Klasse mikroporöser Festkörper bilden die Alumophosphate, Silicoalumophosphate und Metallalumophosphate.

Die synthetischen Zeolithe werden nach Porenweiten (meist noch in Angström-Einheit) in eng-, mittel- u. weitporige Typen eingeteilt. Innerhalb dieser Gruppe existieren mehr als 150 verschiedene Strukturen, die sich häufig im $SiO_2$/$Al_2O_3$-Verhältnis (dem sogenannten Modul) unterscheiden lassen.

Allgemein werden synthetische Zeolithe vorwiegend mit Trivialnamen wie zum Beispiel Zeolith A, X, Y, L, b u. a. belegt oder auch als ZSM-Typen u. a. bezeichnet. Zur Charakterisierung werden v. a. Röntgendiffraktometrie, Festkörper-NMR, FT-IR-Spektroskopie, Thermoanalysen, Elektronenmikroskopie, Adsorptionsmessungen und katalytische Reaktionen herangezogen.

Gemäß JP 60 181 002 werden die antibakteriellen Zeolithe aus natürlichem oder synthetischem Zeolith als Träger und aus mindestens einem ionenaustauschbaren Metall-Ion aus der Gruppe Silber, Kupfer und Zink durch Substitution beispielsweise in Wasser und unter Verwendung eines anorganischen oder organischen Bindemittels hergestellt. Nach anschließender Trocknung wird das Produkt in einem Bereich unter der Temperatur, bei der eine Zersetzung des Zeoliths einsetzt, bei normalem Druck oder Unterdruck gebrannt. Die antibakteriellen Zeolithe enthalten 0,0006 bis 4 % Silber, 0,03 bis 10 % Kupfer oder 0,04 bis 14 % Zink.

Erfindungsgemäß besonders hervorzuheben ist die neuartige Verwendung der Zeolithe als Bestandteil in einer selbstklebenden Polyurethanmatrix, die als hydroaktiven Wundauflage für die feuchte Wundheilung eingesetzt werden kann.

Vorzugsweise kommen elastische, vernetzte Polyurethane mit einem Masseauftragsgewicht von 50 bis 2500 $g/m^2$ zum Einsatz, wie sie zum Beispiel in der WO 97/43328 A1 beschrieben sind.

Gegenstand der Erfindung sind dort hydrophile, selbstklebende Polyurethan-Gele, die aus folgender Zusammensetzung bestehen:

> a) 2 bis 6 Hydroxylgruppen aufweisende Polyetherpolyole mit OH-Zahlen von 20 bis 112 und einem Ethylenoxid (EO)-Gehalt von >= 10 Gew.-%,
> b) Antioxydantien,
> c) in den Polyolen a) lösliche Wismut-(HI)-Carboxylate auf Basis von Carbonsäuren mit 2 bis 18 C-Atomen als Katalysatoren sowie
> d) Hexamethylendiisocyanat oder modifiziertem Hexamethylendiisocyanat, mit einem Produkt der Funktionalitäten der Polyurethan bildenden Komponenten a) und d) von mindestens 5,2, wobei die Katalysatormenge c) 0,005 bis 0,25 Gew.-%, bezogen auf das Polyol a) beträgt, die Menge an Antioxidantien b) im Bereich von 0,1 bis 1,0 Gew.-%, bezogen auf Polyol a) liegt und ein Verhältnis von freien NCO-Gruppen der Komponente d) zu den freien OH-Gruppen der Komponente a) (Isocyanatkennzahl) im Bereich von 0,30 bis 0,70 gewählt wird.

Bevorzugt werden 3 bis 4, ganz besonders bevorzugt 4-Hydroxylgruppen, aufweisende Polyetherpolyole eingesetzt mit einer OH-Zahl im Bereich von 20 bis 112, bevorzugt 30 bis 56. Der Ethylenoxidgehalt liegt bei den eingesetzten Polyetherpolyolen bei vorzugsweise >= 20 Gew.-%.

Die Polyetherpolyole sind als solche an sich bekannt und werden zum Beispiel durch Polymerisation von Epoxiden, wie Ethylenoxid, Propylenoxid, Butylenoxid oder Tetrahydrofuran, mit sich selbst oder durch Anlagerung dieser Epoxide, vorzugsweise von Ethylenoxid und Propylenoxid - gegebenenfalls im Gemisch untereinander oder separat nacheinander - an Starterkomponenten mit mindestens zwei reaktionsfähigen Wasserstoffatomen, wie Wasser, Ethylenglykol, Propylenglykol, Diethylenglykol, Dipropylenglykol, Glyzerin, Trimethylolpropan, Pentaerythrit, Sorbit oder Succrose, hergestellt. Vertreter der genannten, zu verwendenden höhermolekularen Polyhydroxylverbindungen sind zum Beispiel in High Polymers, Vol. XVI, ,"Polyurethanes, Chemistry and Technology" (Saunders-Frisch, Interscience Publishers, New York, Bd 1, 1962, S. 32-42) aufgeführt.

Als Isocyanatkomponente wird monomeres oder trimerisiertes Hexamethylendiisocyanat oder durch Biuret-, Uretdion-, Allophanatgruppen oder durch Prepolymerisierung mit Polyetherpolyolen oder Mischungen von Polyetherpolyolen auf Basis der bekannten Starterkomponenten mit 2 oder > 2 reaktionsfähigen H-Atomen und Epoxyden, wie Ethylenoxid oder Propylenoxid einer OH-Zahl von <= 850, bevorzugt 100 bis 600, modifiziertes Hexamethylendiisocyanat eingesetzt. Bevorzugt ist der Einsatz von modifiziertem Hexamethylendiisocyanat, insbesondere durch Prepolymerisierung mit Polyetherdiolen der OH-Zahl 200 bis 600 modifiziertes Hexamethylendiisocyanat. Ganz besonders bevorzugt sind Modifizierungen des Hexamethylendiisocyanats mit Polyetherdiolen der OH-Zahl 200 bis 600, deren Restgehalt an monomeren Hexamethylendiisocyanat unter 0,5 Gew.-% liegt.

Als Antioxidantien kommen für die Polyurethan-Gele insbesondere sterisch gehinderte phenolische Stabilisatoren, wie BHT (2,6-Di-tert.butyl-4-methylphenol), Vulkanox BKF (2,2 min -Methylen-bis-(6-tert.-butyl-4-methyl phenol) (Bayer AG), Irganox 1010 (Pentaerythrityl-tetrakis-[3-(3,5-ditert.-butyl-4- hydroxyphenyl)-propionat]), Irganox 1076 (Octadecyl-3-(3,5-ditert.-butyl-4- hydroxyphenyl)-propionat) (Ciba-Geigy) oder Tocopherol (Vitamin E) in Betracht. Bevorzugt werden solche vom Typ des a-Tocopherol eingesetzt. Die Antioxidantien werden bevorzugt in Mengen von 0,15 bis 0,5 Gew.-%, bezogen auf das Polyol a), eingesetzt.

Die Polyurethan-Gelmassen werden hergestellt nach üblichen Verfahren, wie sie beispielsweise beschrieben sind in Becker/Braun, Kunststoff- Handbuch, Bd. 7, Polyurethane, S. 121 ff, Carl-Hauser, 1983.

Weiterhin kommen elastische, thermoplastische Polyurethane zum Einsatz, wie sie in der DE-C 19 34 710 beschrieben sind und die sich durch eine gute Hautverträglichkeit sowie Sauerstoff- und Wasserdampfdurchlässigkeit auszeichnen. Besonders vorteilhaft haben sich aliphatische Polyesterurethane erwiesen.

Eine hiermit hergestellte Wundauflage ist vorzugsweise etwa 30 bis 40 μm stark, transparent, weist eine Reißdehnung von über 450% und eine Wasserdampfdurchlässigkeit von über 500 g/m$^2$ in 24h bei 38 °C und 95% rel. Feuchte nach DAB auf.

Daneben lassen sich jedoch auch Wundauflagen auf anderer Grundlage, wie zum Beispiel Acrylat-Copolymere oder die anderen bekannten filmbildenden elastischen Polymeren, verwenden.

Die Dicke der Wundauflagen kann dabei zwischen etwa 15 bis 300, vorzugsweise 15 bis 80 μm, das Gewicht entsprechend zwischen etwa 15 bis 350 g/m$^2$, vorzugsweise 15 bis 100 g/m$^2$, die Höchstzugkraft längs zwischen etwa 5 bis 100 N/cm, vorzugsweise 2 bis 40 N/cm, und die Reißdehnung längs zwischen etwa 100 bis 1000% betragen.

Überraschend wurde gefunden, daß sich die Zeolithe in Polyurethane durch Zumischen des Zeoliths zu den Polyurethanrohstoffen ohne Störung der Reaktion einarbeiten lassen und ihre antimikrobielle Wirkung trotz Einbindung in das Polymer entfalten können.

Zusätzlich ist die Einarbeitung von Superabsorberpulvern, beispielsweise von Favor® (Stockhausen), zum Zweck der selektiven Aufnahme von Wasser möglich.

Weitere Anwendungen ergeben sich durch die Schäumung der beschriebenen Polyurethane. Die ungeschäumten oder geschäumten Massen können flächig mit unterschiedlichen Masseauftragsgewichten ausgestrichen und optional an der hautabgewandten Seite mit einer Folie beispielsweise aus Polyethylen, Polyurethan, Polyester oder Folien/Vliesverbundmaterialien abgedeckt werden.

Gegebenenfalls wird die Wundauflage auf der hautzugewandten Seite mit einer selbstklebenden Beschichtung versehen, die bevorzugt partiell aufgetragen wird.

Eine geeignete Klebemasse ist in der Schrift DE 27 43 979 C3 dargelegt, weiterhin sind für die Klebebeschichtung bevorzugt handelsübliche druckempfindliche Klebmassen auf Acrylat- oder Kautschukbasis einsetzbar.

Besonders bevorzugt werden thermoplastische Heißschmelzklebemassen auf Basis natürlicher und synthetischer Kautschuke und anderer synthetischer Polymere wie Acrylate, Methacrylate, Polyurethane, Polyolefine, Polyvinylderivate, Polyester oder Silikone mit entsprechenden Zusatzstoffen wie Klebharzen, Weichmachern, Stabilisatoren und anderen Hilfsstoffen soweit erforderlich.

Gegebenenfalls kann eine Nachvernetzung durch UV- oder Elektronenstrahlen-Bestrahlung angebracht sein. Insbesondere Heißschmelzklebemassen auf Basis von Blockcopolymeren zeichnen sich durch ihre vielfältige Variationsmöglichkeiten aus, denn durch die gezielte Absenkung der Glasübergangstemperatur der Selbstklebemasse infolge der Auswahl der Klebrigmacher, der Weichmacher sowie der Polymermolekülgröße und der Molekularverteilung der Einsatzkomponenten wird die notwendige funktionsgerechte Verklebung mit der Haut auch an kritischen Stellen des menschlichen Bewegungsapparates gewährleistet. Ihr Erweichungspunkt sollte höher als 50 °C liegen, da die Applikationstemperatur in der Regel mindestens 90 °C beträgt, bevorzugt zwischen 120 °C und 150 °C beziehungsweise 180 °C und 220 °C bei Silikonen.

Die hohe Scherfestigkeit der Heißschmelzklebemasse wird durch die hohe Kohäsivität des Polymeren erreicht. Die gute Anfaßklebrigkeit ergibt sich durch die eingesetzte Palette an Klebrigmachern und Weichmachern.

Die Klebemasse beinhaltet vorzugsweise mindestens eine aromatische Komponente, welche einen Anteil von weniger als 35 %, bevorzugt 5 bis 30 %, aufweist.

Für besonders starkklebende Systeme basiert die Heißschmelzklebemasse bevorzugt auf Blockcopolymeren, insbesondere A-B-, A-B-A-Blockcopolymere oder deren Mischungen. Die harte Phase A ist vornehmlich Polystyrol oder dessen Derivate, und die weiche Phase B enthält Ethylen, Propylen, Butylen, Butadien, Isopren oder deren Mischungen, hierbei besonders bevorzugt Ethylen und Butylen oder deren Mischungen.

Insbesondere die gezielte Abmischung von Di-Block- und Tri-Blockcopolymeren ist vorteilhaft, wobei ein Anteil an Di-Blockcopolymeren von kleiner 80 Gew.-% bevorzugt wird. In einer vorteilhaften Ausführung weist die Heißschmelzklebemasse die nachfolgend angegebene Zusammensetzung auf:

| 10 Gew.-% bis 90 Gew.-% | Blockcopolymere, |
| --- | --- |
| 5 Gew.-% bis 80 Gew.-% | Klebrigmacher wie Öle, Wachse, Harze und/oder deren Mischungen, bevorzugt Mischungen aus Harzen und Ölen, |
| weniger als 60 Gew.-% | Weichmacher, |
| weniger als 15 Gew.-% | Additive, |
| weniger als 5 Gew.-% | Stabilisatoren. |

Die als Klebrigmacher dienenden aliphatischen oder aromatischen Öle, Wachse und Harze sind bevorzugt Kohlenwasserstofföle, -wachse und -harze, wobei sich die Öle, wie Paraffinkohlenwasserstofföle, oder die Wachse, wie Paraffinkohlenwasserstoffwachse, durch ihre Konsistenz günstig auf die Hautverklebung auswirken. Als Weichmacher finden mittel- oder langkettige Fettsäuren und/oder deren Ester Verwendung. Diese Zusätze dienen dabei der Einstellung der Klebeeigenschaften und der Stabilität. Gegebenenfalls kommen weitere Stabilisatoren und andere Hilfsstoffe zum Einsatz.

Eine Silberzeolithpartikel enthaltende Wundauflage wird nach Applikation auf eine exsudierende Wunde durch den Kontakt von Flüssigkeit mit den Silberzeolithpartikeln die in der Wundflüssigkeit befindenden Keime abtöten, beziehungsweise eine Kolonisation und unter Umständen eine Infektion der Wunde mit Mikroorganismen unterbinden. Mit dem Entfernen der Silberzeolithpartikel enthaltenden Wundauflage ist die antibakterielle Wirkung aufgehoben. Ein Nachwaschen der Wunde zum Entfernen von zuvor temporär applizierten Antiseptika und Antibiotika entfällt.

Bevorzugt werden Zeolithpartikel eingesetzt, die neben einer Freisetzung von Silberlonen auch Zink-lonen enthalten. Dabei werden im feuchten Milieu durch die lonenaustauscherwirkung des Zeoliths geringe, definierte Mengen an Silber- und Zink-lonen freigesetzt, so daß eine langanhaltende antibakterielle Wirkung garantiert wird.

Die beschriebene Erfindung basiert somit auf der bereits beschriebenen antimikrobiellen Wirkung von silberdotierten Zeolithpartikeln in Kombination mit einer stark saugenden Wundauflage, die zusammen einen synergetischen Effekt erreichen. Ferner kann eine Wundauflage, wie etwa eine Polyurethan-Wundauflage über selbstklebende Eigenschaften verfügen, die eine Fixierung auf der intakten Haut am Wundrand des Patienten ermöglichen und eine Complience erzeugen. Sie betrifft eine neuartige Wundauflage, die zur Behandlung infizierter Wunden oder zum vorbeugenden Schutz vor Wundinfektionen eingesetzt werden können. Dabei bildet die Wundauflage eine Barriere für Mikroorganismen, die das Eindringen von außen verhindert, indem diese beim Kontakt mit der antimikrobiellen Wundauflage abgetötet werden.

Erfindungsgemäße Wundauflagen werden nachfolgend in bevorzugter Ausführung anhand mehrerer Beispiele beschrieben, ohne damit die Erfindung in irgendeiner Weise beschränken zu wollen. Des weiteren ist ein Vergleichsbeispiel aufgeführt.

**Beispiele**

Für die im folgenden beschriebenen Versuche wird ein silberionenhaltiges Zeolith der Firma Shinanen (Handelsbezeichnung ‚Antimicrobial Zeomic") mit einer mittleren Partikelgröße von 0,6 bis 2,5 µm verwendet.

**Beispiel 1 (Vergleich)**

Es wurden 29,8 g Favor (teilneutralisierte Polyacrylsäure der Fa. Stockhausen, Krefeld) in 63,8 g Levagel (Polyetherpolyol der Fa. Bayer, Leverkusen) über eine Stunde dispergiert. Anschließend wurde die Dispersion mit 6,2 g Desmodur (Polyisocyanat auf Hexamethylendiisocyanat-Basis der Bayer, Leverkusen) und 0,50 g Coscat 83 (Bismut-Salz der Fa. C.H.Erbslöh) homogen vermischt und die noch flüssige Masse zwischen einem Polyurethanträger (Beiersdorf, Hamburg) und einem Silikonpapier bei einer Spaltbreite von 1,2 mm flächig ausgestrichen. Die Vernetzungszeit der Polyurethanmasse beträgt 4 min 30 sec.

**Beispiel 2**

Es wurden 27,3 g Favor in 63,7 g Levagel über eine Stunde dispergiert. Anschließend wurde die Dispersion mit 5,7 g Desmodur, 2,8 g Zeomic (Silberzinkzeolith mit ca. 2,2% Silber und ca. 12,5 % Zink der Fa. Shinanen, Japan) und 0,5 g Coscat 83 homogen vermischt und die noch flüssige Masse zwischen einem Polyurethanträger und einem Silikonpapier bei einer Spaltbreite von 1,2 mm flächig ausgestrichen. Die Vernetzungszeit der Polyurethanmasse beträgt 4 min 50 sec.

**Beispiel 3**

Es wurden 24,6 g Favor in 57,4 g Levagel über eine Stunde dispergiert. Anschließend wurde die Dispersion mit 5,1 g Desmodur, 12,5 g Zeomic und 0,4 g Coscat 83 homogen vermischt und die noch flüssige Masse zwischen einem Polyurethanträger und einem Silikonpapier bei einer Spaltbreite von 1,2 mm flächig ausgestrichen. Die Vemetzungszeit der Polyurethanmasse beträgt 5 min.

**Beispiel 4**

Es wurden 87,1 g Levagel, 8,5 g Desmodur, 3,8 g Zeomic und 0,6 g Coscat 83 homogen vermischt und die noch flüssige Masse zwischen einem Polyurethanträger und einem Silikonpapier bei einer Spaltbreite von 1,2 mm flächig ausgestrichen. Die Vernetzungszeit der Polyurethanmasse beträgt 4 min 30 sec.

**Untersuchungen zur antimikrobiellen Aktivität von Silberzeolithen**

Bakterizide Wirkung von Siberzeolithen im Quantitativen Suspensionsversuch nach DGHM* Borneff et al. (1981) Zbl. Bakt. Hyg. Reihe B: Band 172, Heft 6
Beschreibung des Verfahrens:

- Bestimmung der Inaktivierungsmittelkombination nach DGHM (Deutsche Gesellschaft für Hygiene und Mikrobiologie).

- Die mikrobizide Wirkung der Silberzeolithe wird bis zu einer Konzentration von 0,5% (w/v) durch die Kombination TLHC (Tween 3%, Lecithin 0.3%, Histidin 0.1% Cystein 0.1%) inaktiviert.

Testdurchführung:

Die Teststämme wurden als Übemachtkultur in Caso- beziehungsweise Sabouraud-bouillon angezogen. 0,1 ml dieser Keimkulturen wurden jeweils in einem sterilen Reagenzglas mit 10 ml einer wäßrigen 0,5% (w/v) Silberzeolithsuspension versetzt.
Parallel wurde zur Ermittlung eines Kontrollwerts ebenfalls 0,1 ml der Keimkulturen in jeweils einem weiteren sterilen Reagenzglas mit 10 ml sterilem VE-Wasser versetzt. Die Einwirkzeit auf die Teststämme betrug 1h bei Raumtemperatur.
Nach Ablauf der Einwirkzeit wurde dem Silberzeoliht-Keim-Gemisch 1 ml entnommen und in 9 ml Inaktivierungsmittelflüssigkeit überführt. Nach längstens 30 min. Kontaktzeit in dieser Lösung wurden weitere geometrische Verdünnungen angelegt. Aus geeigneten Verdünnungen wurden zur Keimzahlbestimmung Gußplatten angelegt bei einer Inkubation von 48 h bis 72 h bei 37 °C.
Mit dem Kontrollwert wurde parallel zur Probe ebenso verfahren.
Für jeden Testkeim ergaben sich zwei Keimzahlen :

1. KBE (Probe = silberzeolithhaltig)
2. KBE (Kontrolle)

(KBE=koloniebildende Einheit)

Der Reduktionsfaktor (RF) berechnet sich für jeden Teststamm nach folgender Formel:

$$\log RF = \log KBE \text{ (Kontrolle)} - \log KBE \text{ (Probe)}$$

Ermittelte Reduktionsfaktoren aus 3 quantitativen Suspensionsversuchen nach DGHM.

| Teststamm Nummer der Labordokumentation | RF (X/S.121-124/Ti) | RF (XXIII/S 76-79/Sch) | RF (XXIV/S.45-48;51+52/Sch) |
|---|---|---|---|
| S.aureus ATCC 6538- | 1,38 | 1,78 | 1,48 |
| E.coli ATCC 11229 | 3,72 | 2,62 | 3,94 |
| Ps.Aeruginosa ATCC 15442 | 5,65 | 3,39 | 6,89 |
| Pr.mirabilis ATCC 14153 | - | 2,39 | 3,33 |
| C.albicans ATCC 10231 | 4,68 | 4,75 | 6,15 |

Erläuterungen zu den Keimen

S.aureus = Staphyloccoccus aureus
E.coli = Escherichia coli
Ps.Aeruginosa = Pseudomonas aeruginosa
Pr.mirabilis = Proteus mirabilis
C.albicans = Candida albicans

Antimikrobielle Wirksamkeit verschiedener Produktmuster

Versuchsaufbau:

Die Anzucht der Teststämme erfolgte als Übernachtkultur in Caso- beziehungsweise Sabouraud-bouillon. Die Teststämme wurden nach Anzucht bei 3500 U/min abzentrifugiert und zweimal mit sterilem VE-Wasser gewaschen. Die Wiederaufnahme der Teststämme erfolgte in sterilem VE-Wasser.

Die γ-sterilisierten Prüfmuster wurden auf der wundzugewandten Seite mit 2 x 50 μl der jeweiligen Keimsuspension kontaminiert und anschließend 1 h bei 32 °C in einer feuchten Kammer inkubiert. Als Kontrolle dienten Muster ohne Silberzeolith (Beispiel 1).

Nach Ablauf der Inkubationszeit wurden die Muster in 100 ml Aufarbeitungslösung überführt, in der sie 5 min zum Quellen ruhten.

Anschließend wurden die Muster 60 sec gestomachert und die Zahl der teilungsfähigen Keime mittels Gußplatte bestimmt. Inkubation: 48 h bis 72 h, 32 °C.

Der Reduktionsfaktor( RF) berechnet sich für jeden Teststamm nach folgender Formel:

$$\log RF = \log KBE \text{ (Kontrolle)} - \log KBE \text{ (Probe)}$$

| Testmuster | Ps. aeruginosa ATCC 15442 | C.albicans ATCC 10231 |
|---|---|---|
| Beispiel 2 2,8% Zeolith, 27,3% Favor | 2,43 | 4,60 (XXV/S.120-123/Sch) |
| Beispiel 3 12,5 %Zeolith, 24,6% Favor | 4,07 | 4,60 (XXV/S.120-123/Sch) |
| ATCC : American Type Culture Collection (Angabe der genauen Nummer des verwendeten Testorganismus) | | |

**Patentansprüche**

1. Wundauflage, dadurch gekennzeichnet, daß sie aus einem synthetischen Polymermaterial besteht, das metallionenhaltige Zeolithe enthält.

2. Wundauflage nach Anspruch 1, dadurch gekennzeichnet, daß die metallionenhaltigen Zeolithe zu 0,01 bis 40 Gew.-%, bevorzugt 0,1 bis 6 Gew.-%, in dem synthetischen Polymermaterial enthalten sind.

3. Wundauflage nach Anspruch 1, dadurch gekennzeichnet, daß das synthetische Polymermaterial gewählt ist aus der Gruppe Polyacrylate, SIBS-Massen, SEBS-Massen, Naturkautschukmassen, Chitosane, Alginate, Hydrogele, Hydrokolloide, insbesondere Polyurethane.

4. Wundauflage nach Anspruch 1, dadurch gekennzeichnet, daß das synthetische Polymermaterial geschäumt ist.

5. Wundauflage nach Anspruch 1, dadurch gekennzeichnet, daß das synthetische Polymermaterial an der hautabgewandten Seite mit einer Folie und/oder mit einem FolienNliesverbundmaterial abgedeckt ist.

6. Wundauflage nach Anspruch 1, dadurch gekennzeichnet, daß zusätzlich Superabsorber enthalten sind.